# EUROPEAN PATENT APPLICATION

(11) **EP 3 640 276 A1**
(43) Date of publication of application: **22.04.2020**
(21) Application number: 18200769.0
(22) Date of filing: 16.10.2018
(51) Int. Cl.: C08G 59/24, C08G 59/26, C08G 59/40

(54) **HIGH HEAT EPOXY-TERMINATED POLYOXAZOLIDONES, COMPOSITIONS, METHODS OF MANUFACTURE, AND USES THEREOF**

(71) Applicant: SABIC Global Technologies B.V., 4612 PX Bergen op Zoom (NL)
(72) Inventor: Pal, Tarun Kumar, 562125 Bangalore (IN); Sista, Prakash, Mt Vernon, 47620 (US); Pal, Jaykisor, 562125 Bangalore (IN)
(74) Representative: Balder IP Law, S.L.

(57) **Abstract**

A thermosetting oligomer is disclosed, comprising repeat units of the formula 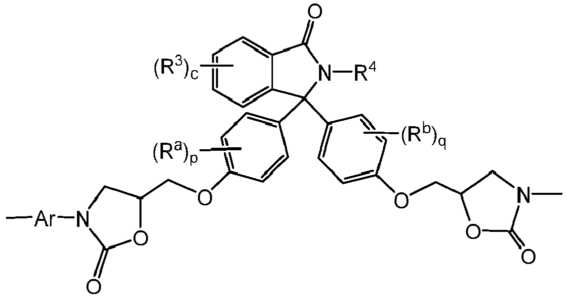 wherein
Ar is a C₆₋₃₂ arylene, preferably a C₁₃₋₁₇ diarylene methylene;
R^{a} and R^{b} at each occurrence are each independently a halogen, C₁₋₁₂ alkyl, C₂₋₁₂ alkenyl, C₃₋₈ cycloalkyl, or C₁₋₁₂ alkoxy, preferably a C₁₋₃ alkyl;
R³ at each occurrence is independently a halogen or a C₁₋₆ alkyl group, preferably a C₁₋₃ alkyl;
R⁴ a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; and
c, p and q at each occurrence are each independently 0 to 4.

## Description

### BACKGROUND

Epoxy thermosets are formed from thermosetting epoxy resin compositions containing multifunctional epoxy oligomers. The multifunctional epoxy oligomers can in turn be formed from one or more multifunctional epoxide compounds. Use of oligomers allows for the growth of the linear molecular weight of the thermoset polymers. The oligomers can be formed, for example cast, into a specific shape and permanently hardened (cured) at a certain temperature to provide the epoxy thermosets. In their cured form, epoxy resins offer desirable properties including good adhesion to other materials, excellent resistance to corrosion and chemicals, high tensile strength, and good electrical resistance.

Despite the wide variety of epoxy resins available for use, there remains a need for thermosetting epoxy oligomers that provide thermosets with high thermal stability. It would be a further advantage if such compositions had lower viscosity, allowing easier processing.

### SUMMARY

A thermosetting oligomer is provided, comprising repeat units of the formula 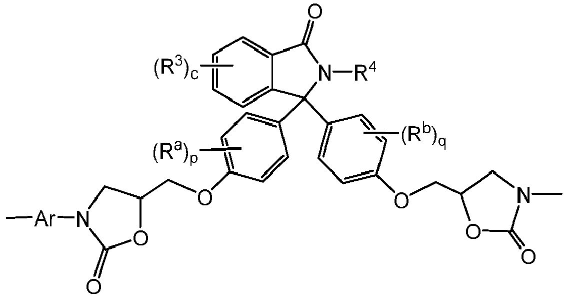 wherein Ar is a C₆₋₃₂ arylene, preferably a C₁₃₋₁₇ diarylene methylene; R^{a} and R^{b} at each occurrence are each independently a halogen, C₁₋₁₂ alkyl, C₂₋₁₂ alkenyl, C₃₋₈ cycloalkyl, or C₁₋₁₂ alkoxy, preferably a C₁₋₃ alkyl; R³ at each occurrence is independently a halogen or a C₁₋₆ alkyl group, preferably a C₁₋₃ alkyl; R⁴ a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; and c, p and q at each occurrence are each independently 0 to 4.

A thermosetting composition comprises the above-described thermosetting oligomer, a curing promoter, optionally a catalyst, and optionally, an auxiliary compound co-curable with the thermoset oligomer.

Also disclosed is a thermoset comprising the cured thermosetting composition.

Articles comprising the thermoset are also disclosed.

The invention is further illustrated by the following figures, detailed description, examples, and claims.

### BRIEF DESCRIPTION OF THE DRAWINGS

The following Figures are exemplary.
FIG. 1 shows an infrared spectrum for Example 1.
FIG. 2 shows a comparison of the ¹H NMR spectra of the PPP-BP oxazolidone (top) with the PPP-BP epoxy (bottom).
FIG. 3 shows the ¹H NMR spectrum of Example 2.

### DETAILED DESCRIPTION

Disclosed herein are high heat epoxy polyoxazolidone oligomers, particularly epoxy phthalimidine-polyoxazolidone oligomers, processes for making the high heat epoxy oligomers, thermosetting compositions comprising the same, thermosets prepared from the thermosetting compositions, and articles made from the thermosets. The processes for making the high heat epoxy oligomers can result in oligomers of high purity. Certain epoxy phthalimidine-polyoxazolidone oligomers can further have low viscosity, rendering them more processable than higher viscosity epoxy resins.

The thermosetting phthalimidine-polyoxazolidone oligomer comprises units of formula (1) 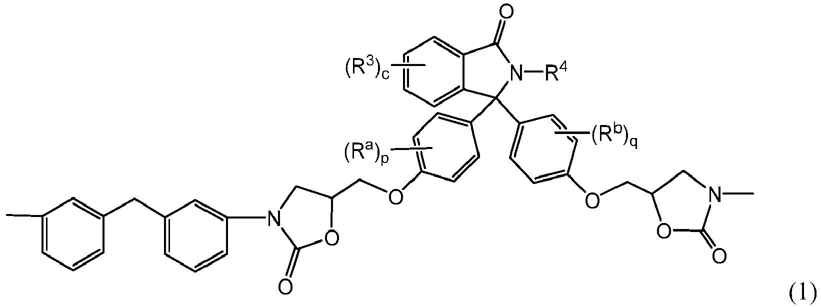
wherein R^{a} and R^{b} at each occurrence are each independently a halogen, C₁₋₁₂ alkyl, C₂₋₁₂ alkenyl, C₃₋₈ cycloalkyl, or C₁₋₁₂ alkoxy; R³ at each occurrence is independently a halogen or a C₁₋₆ alkyl group;
R⁴ is a C₁₋₂₅ hydrocarbyl or a phenyl substituted with up to five C₁₋₆ alkyl groups; and c, p and q at each occurrence are each independently 0 to 4.

In an aspect of formula (1), R^{a} and R^{b} at each occurrence are each independently a C₁₋₃ alkyl; R³ at each occurrence is independently a C₁₋₃ alkyl; R⁴ is a C₁₋₃ alkyl or a phenyl; and c, p and q at each occurrence are each independently 0 or 1.

A more preferred aspect is a thermosetting oligomer, comprising units of formula (1a) 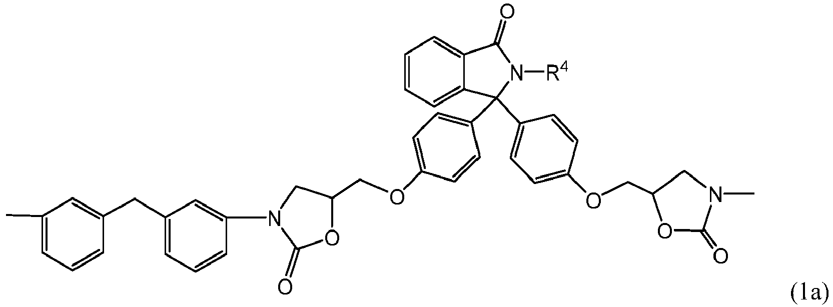 wherein R⁴ is methyl or phenyl. Preferably in formula (1a), R is phenyl.

The thermosetting phthalimidine-polyoxazolidone oligomer of formula (1) and formula (1a) is epoxy-group terminated. In an embodiment, the thermosetting oligomer is a diepoxy, i.e., is terminated with two epoxy groups. Each epoxy at each occurrence can independently be of formula (2) 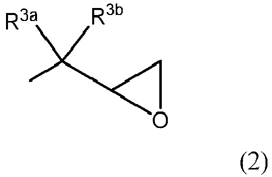 wherein R^{3a} and R^{3b} are each independently hydrogen or C₁₋₁₂ alkyl, preferably hydrogen. Thus in a preferred embodiment the epoxy group is of formula (2a).

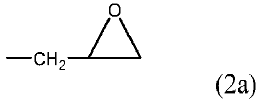

The epoxy groups can arise from an unreacted epoxy group of the diepoxy phthalimidine of formula (3) as described in further detail below. It is to be understood, however, that a portion of the oligomers may be mono-epoxy-terminated, or terminated with a different group, for example a hydroxyl, cyano, or amino group. Preferably, at least 80%, at least 90%, or at least 95% of the thermosetting oligomer is a diepoxy oligomer.

The term "oligomer" is used herein for convenience, and means that a plurality of the repeat units of formulas (1) or (1a) is present. In an embodiment, the number of repeat units is 2 to 50, or 2 to 40, or 2 to 30, or 2 to 20, or 2 to 10.

A method for the manufacture of a thermosetting oligomer includes reacting an arylene polyisocyanate and a diepoxy phthalimidine in the presence of a catalyst, and optionally, a solvent.

In an embodiment, the arylene polyisocyanate is an arylene diisocyanate. Use of an arylene polyisocyanate having higher functionality (for example 3, 4, or 5 isocyanate groups) provides a branched thermosetting oligomer. Examples of specific arylene polyisocyanates include methylene diphenyl isocyanate, toluene diisocyanate, hexamethylene diisocyanate, and isophorone diisocyanate. In an embodiment the polyisocyanate is methylene diphenyl isocyanate.

The diepoxy phthalimidine is of formula (3) 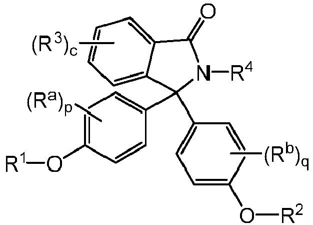 wherein R¹ and R² are each independently an epoxide-containing functional group; R^{a} and R^{b} at each occurrence are each independently a halogen, C₁₋₁₂ alkyl, C₂₋₁₂ alkenyl, C₃₋₈ cycloalkyl, or C₁₋₁₂ alkoxy; R³ at each occurrence is independently a halogen or a C₁₋₆ alkyl group R⁴ a C₁₋₂₅ hydrocarbyl, a phenyl, or a phenyl substituted with up to five C₁₋₆ alkyl groups; and c, p and q at each occurrence are each independently 0 to 4.

In an aspect of formula (3), R¹ and R² at each occurrence are each independently of formula (2) 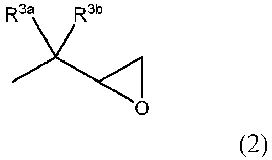 wherein R^{3a} and R^{3b} are each independently hydrogen or C₁₋₁₂ alkyl, preferably hydrogen; R^{a} and R^{b} at each occurrence are each independently a C₁₋₃ alkyl; R³ at each occurrence is independently a C₁₋₃ alkyl; R⁴ is a C₁₋₃ alkyl or a phenyl; and c, p and q at each occurrence are each independently 0 or 1.

Preferably, the diepoxy phthalimidine is of formula (3) wherein R¹ and R² are each of formula (2a) 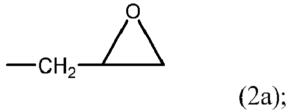 R⁴ is a methyl or a phenyl, preferably phenyl; and c, p, and q at each occurrence are each 0.

In the reaction, a mole ratio of the arylene polyisocyanate to the diepoxy phthalimidine can be from 1:1 to 1:2.

The reaction is in the presence of a catalyst, which can be a phase transfer catalyst. For example, the catalyst can be an ammonium or phosphonium catalyst, such as quaternary compounds of the formula (R³)₄Q⁺X, wherein each R³ is the same or different, and is a C₁₋₁₀ alkyl; Q is a nitrogen or phosphorus atom; and X is a halogen atom or a C₁₋₈ alkoxy or C₆₋₁₈ aryloxy. Exemplary phase transfer catalysts include (CH₃(CH₂)₃)₄NX, (CH₃(CH₂)₃)₄PX, (CH₃(CH₂)₅)₄NX, (CH₃(CH₂)₆)₄NX, (CH₃(CH₂)₄)₄NX, CH₃(CH₃(CH₂)₃)₃NX, and CH₃(CH₃(CH₂)₂)₃NX, wherein X is Cl⁻, Br⁻, a C₁₋₈ alkoxy or a C₆₋₁₈ aryloxy. Quaternary ammonium salts are preferred, such as tetrabutylammonium bromide, methyltrioctylammonium chloride, and methyltrialkyl(C₈-C₁₀)ammonium chloride. Other phase transfer catalysts include guanidinium salts, such as hexaethylguanidinium chloride (HEGCl) hexa-n-propylguanidinium chloride (HPGCl), or the like. Suitable amounts of catalyst include 1 to 60 mole percent based on the moles of bisphenol of formula (2), preferably 1 to 40 mole percent, preferably 1 to 20 mole percent, preferably 1 to 10 mole percent, and preferably 1 to 5 mole percent.

An organic solvent can optionally be present, and is used especially when the diepoxy phthalimidine (3) is not a liquid at the reaction temperature. Preferred solvents are polar organic solvents such as N-methyl pyrrolidone, tetrahydrofuran, ethyl acetate, acetone, N,N-dimethylformamide, acetonitrile, dimethyl sulfoxide, nitromethane, or the like.

The reaction can be conducted at elevated temperature and atmospheric pressure. For example, the reaction can be conducted at 120 to 250°C, or at 150 to 200°C, at atmospheric pressure. Other conditions can be used to increase the rate of the reaction, or to minimize side products.

The diepoxy phthalimidine can have a purity of greater than 60%, preferably greater than 70%, preferably greater than 80%, preferably greater than 90%, preferably greater than 95%, preferably greater than 98%, and preferably greater than 99% as determined by high performance liquid chromatography.

The diepoxy phthalimidine can have an epoxy equivalent weight of 1.0 to 1.5, preferably 1.0 to 1.2, more preferably 1.0 to 1.15, even more preferably 1.0 to 1.1 times relative to the theoretical epoxy equivalent weight.

The diepoxy phthalimidine can have a metal impurity content of 3 ppm or less, 2 ppm or less, 1 ppm or less, 500 ppb or less, 400 ppb or less, 300 ppb or less, 200 ppb or less, or 100 ppb or less. The metal impurities may be iron, calcium, zinc, aluminum, or a combination thereof. The diepoxy phthalimidine can have an unknown impurities content of 0.1 weight percent or less.

The diepoxy phthalimidine can have a color APHA value of 40 or less, 35 or less, 30 or less, 25 or less, 20 or less, 19 or less, 18 or less, 17 or less, 16 or less, or 15 or less, as measured using test method ASTM D1209.

The polyoxazolidone-phthalimidine oligomers obtained can be useful in thermosetting compositions for the manufacture of thermosets. A thermosetting composition comprises the polyoxazolidone-phthalimidine oligomer of formula (1), preferably of formula (1a); a curing promoter; and optionally, an auxiliary compound co-curable with the thermosetting oligomer.

The term "curing promoter" as used herein encompasses compounds whose roles in curing epoxy compounds are variously described as those of a hardener, a hardening accelerator, a crosslinking agent, a curing catalyst, and a curing co-catalyst, among others. Curing promoters can be either coreactive or catalytic.

Coreactive curing promoters have active hydrogen atoms that can react with epoxy groups of the epoxy resin to form an extended or cross-linked resin. The active hydrogen atoms can be present in functional groups comprising primary or secondary amines, phenols, thiols, carboxylic acids, or carboxylic acid anhydrides. Examples of coreactive curing promoters for epoxy resins include aliphatic and cycloaliphatic amines and amine-functional adducts with epoxy resins, Mannich bases, aromatic amines, polyamides, amidoamines, phenalkamines, dicyandiamide, polycarboxylic acid-functional polyesters, carboxylic acid anhydrides, amine-formaldehyde resins, phenol-formaldehyde resins, polysulfides, polymercaptans, or a combination thereof.

A catalytic curing promoter functions as an initiator for epoxy resin homopolymerization or as an accelerator for coreactive curing agents. Examples of catalytic curing agents include tertiary amines, such as 2-ethyl-4-methylimidazole, Lewis acids, such as boron trifluoride, and latent cationic cure catalysts, such as diaryliodonium salts. In some embodiments both a coreactive curing promoter and a catalytic curing promoter is present in the thermosetting compositions.

In an embodiment, the curing promoter is an amine compound, an aromatic dianhydride, or a bicyclic anhydride. Preferably, the amine compound is a diamine compound, examples of which include 4,4'-diamino diphenyl sulfone, 4,4'-methylenebis-(2,6-diethylaniline), 4,4'-methylenedianiline, diethyltoluenediamine, 4,4'-methylenebis-(2,6-dimethylaniline), m-phenylenediamine, p-phenylenediamine, 2,4-bis(p-aminobenzyl)aniline, 3,5-diethyltoluene-2,4-diamine, 3,5-diethyltoluene-2,6-diamine, m-xylylenediamine, p-xylylenediamine, diethyl toluene diamines, or a combination thereof. Most preferably the curing promoter is 4,4'-diamino diphenyl sulfone, 4,4'-methylenebis-(2,6-diethylaniline), methyl-5-norbornene-2,3-dicarboxylic anhydride, or a combination thereof.

The curing promoter can be an aromatic dianhydride. In aspects, the aromatic dianhydride compound has the general structure 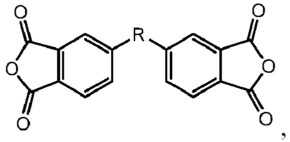 wherein R can be a single bond, 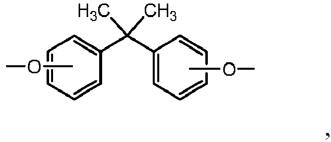 other bisphenols, -C(CF₃)₂-,-O-, or -C(=O)-.

Examples of aromatic dianhydride curing promotors include 4,4'-(4,4'-isopropylidenediphenoxy)bis-(phthalic anhydride) (CAS Reg. No. 38103-06-9), 4,4'-(hexafluoroisopropylidene)diphthalic anhydride (CAS Reg. No. 1107-00-2), 4,4'-oxydiphthalic anhydride (CAS Reg. No. 1823-59-2), benzophenone-3,3',4,4'-tetracarboxylic dianhydride (CAS Reg. No. 2421-28-5), and 3,3',4,4'-biphenyltetracarboxylic dianhydride (CAS Reg. No. 2420-87-3), and specifically compounds having the formulas below.

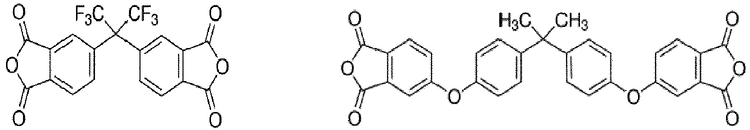

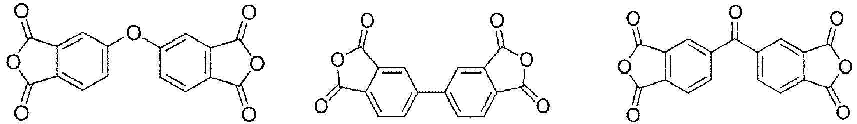

The curing promoter can be a bicyclic anhydride. In aspects, the bicyclic anhydride compound can be methyl-5-norbornene-2,3-dicarboxylic anhydride (CAS Reg. No. 25134-21-8) and cis-5-norbornene-endo-2,3-dicarboxylic anhydride (CAS Reg. No. 129-64-6) and specifically compounds having the formulas below.

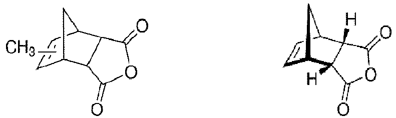

Preferably, the curing promoter is a diamine compound or a bicyclic anhydride, for example 4,4'-diamino diphenyl sulfone, 4,4'-methylenebis-(2,6-diethylaniline), 4,4'-methylenedianiline, diethyltoluenediamine, 4,4'-methylenebis-(2,6-dimethylaniline), m-phenylenediamine, p-phenylenediamine, 2,4-bis(p-aminobenzyl)aniline, 3,5-diethyltoluene-2,4-diamine, 3,5-diethyltoluene-2,6-diamine, m-xylylenediamine, p-xylylenediamine, diethyl toluene diamines, methyl-5-norbornene-2,3-dicarboxylic anhydride, or a combination thereof can be used.

The amount of the curing promoter will depend on the type of curing promoter, as well as the identities and amounts of the other components of the epoxy resin composition. For example, when the curing promoter is an aromatic diamine compound or a bicyclic anhydride, it can be used in an amount of 10 to 45 weight percent of the thermosetting composition, for example 20 to 40 weight percent, based on the thermosetting composition.

Optionally, the thermosetting composition can further include a co-curable auxiliary compound. The co-curable auxiliary compound can comprise an auxiliary epoxy compound, bifunctional epoxy resin, multifunctional epoxy resin, cycloaliphatic epoxy resin, aromatic epoxy resin, or a combination thereof. Preferably the co-curable auxiliary epoxy compound is an aliphatic epoxy compound, cycloaliphatic epoxy compound, aromatic epoxy compound, bisphenol-A epoxy compound, bisphenol-F epoxy compound, phenol novolac epoxy polymer, cresol-novolac epoxy polymer, biphenyl epoxy compound, triglycidyl p-aminophenol, tetraglycidyl diamino diphenyl methane, naphthalene epoxy compound, divinylbenzene dioxide compound, 2-glycidylphenylglycidyl ether, dicyclopentadiene epoxy compound, multi-aromatic epoxy polymer, bisphenol S epoxy compound, isocyanurate epoxy compound, hydantoin epoxy compound, or a combination thereof. More preferably the co-curable auxiliary epoxy compound is a bisphenol-A diglycidylether, a bisphenol-F diglycidylether, a neopentylglycol diglycidyl ether, a 3,4-epoxycyclohexylmethyl-3,4-epoxycyclohexane carboxylate, a *N,N-*diglycidyl-4-glycidyloxyaniline, an N,N,N',N'-tetraglycidyl-4,4'-diaminodiphenylmethane, or a combination thereof.

The thermosetting composition can further comprise an additive. A wide variety of additives as is known in the art can be used, for example a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof. In general, any additive is used in an amount generally known to be effective, which can be from 0.001 to 5 weight percent based on the total weight of the thermosetting composition. For example, the total amount of the additives (other than any filler) can be 0.01 to 10 weight percent, based on the total weight of the thermosetting composition.

In an aspect, the thermosetting composition does not contain a solvent. Alternatively, the thermosetting composition can include a solvent to prepare homogeneous epoxy blends and then the solvent can be removed before or after cure.

A thermoset composition can be obtained by curing, i.e., polymerizing and crosslinking, the thermosetting composition. Cure can be accomplished by subjecting the thermosetting composition to heat. Thus, a method for the manufacture of a thermoset can comprise curing (which can include polymerizing and crosslinking) the thermosetting composition comprising the polyoxazolidone-phthalimidine oligomers described herein. There is no particular limitation on the method by which the thermosetting composition can be cured. The composition can be cured thermally, optionally in conjunction with irradiation techniques, including UV irradiation and electron beam irradiation. When heat curing is used, the temperature can be 80 °C to 300 °C, preferably 120 °C to 240 °C. The heating period can be 1 minute to 10 hours, though such heating period may advantageously be 1 minute to 6 hours, more preferably 3 hours to 5 hours. Such curing may be staged to produce a partially cured and often tack-free resin ("B-staged"), which then is fully cured by heating for longer periods or temperatures within the aforementioned ranges.

The thermosets (the cured compositions) can have a glass transition temperature of greater than or equal to 80°C, preferably greater than or equal to 100°C and more preferably greater than or equal to 140°C.

The thermosets can be used in a variety of forms for various purposes, including a composite (e.g. composite materials such as those using carbon fiber and fiberglass reinforcements), a foam, a fiber, a layer, a coating, an encapsulant, an adhesive, a sealant, a sizing resin, a prepreg, a casing, a component, or a combination comprising one or more of the foregoing. The thermosets can be used to form a number of articles in the aerospace, automobile, and other industries.

In certain aspects, the composite is a glass fiber-containing composite, a carbon fiber-containing composite, or a combination thereof. Methods of forming a composite can include impregnating a reinforcing structure with the thermosetting composition; partially curing the thermosetting composition to form a prepreg; and laminating a plurality of prepregs; wherein the thermosetting composition comprises the polyoxazolidone-phthalimidine oligomer, a curing promoter, optionally, an auxiliary co-monomer, and optionally, one or more additional additives.

Applications for the thermosets obtained from thermosetting oligomer compositions include, for example, acid bath containers; neutralization tanks; aircraft components; bridge beams; bridge deckings; electrolytic cells; exhaust stacks; scrubbers; sporting equipment; stair cases; walkways; automobile exterior panels such as hoods and trunk lids; floor pans; air scoops; pipes and ducts, including heater ducts; industrial fans, fan housings, and blowers; industrial mixers; boat hulls and decks; marine terminal fenders; tiles and coatings; building panels; business machine housings; trays, including cable trays; concrete modifiers; dishwasher and refrigerator parts; electrical encapsulants; electrical panels; tanks, including electro refining tanks, water softener tanks, fuel tanks, and various filament-wound tanks and tank linings; furniture; garage doors; gratings; protective body gear; luggage; outdoor motor vehicles; pressure tanks; printed circuit boards; optical waveguides; radomes; railings; railroad parts such as tank cars; hopper car covers; car doors; truck bed liners; satellite dishes; signs; solar energy panels; telephone switchgear housings; tractor parts; transformer covers; truck parts such as fenders, hoods, bodies, cabs, and beds; insulation for rotating machines including ground insulation, turn insulation, and phase separation insulation; commutators; core insulation and cords and lacing tape; drive shaft couplings; propeller blades; missile components; rocket motor cases; wing sections; sucker rods; fuselage sections; wing skins and flarings; engine nacelles; cargo doors; tennis racquets; golf club shafts; fishing rods; skis and ski poles; bicycle parts; transverse leaf springs; pumps, such as automotive smog pumps; electrical components, embedding, and tooling, such as electrical cable joints; wire windings and densely packed multielement assemblies; sealing of electromechanical devices; battery cases; resistors; fuses and thermal cut-off devices; coatings for printed wiring boards; casting items such as capacitors, transformers, crankcase heaters; small molded electronic parts including coils, capacitors, resistors, and semiconductors; as a replacement for steel in chemical processing, pulp and paper, power generation, and wastewater treatment; scrubbing towers; pultruded parts for structural applications, including structural members, gratings, and safety rails; swimming pools, swimming pool slides, hot-tubs, and saunas; drive shafts for under the hood applications; dry toners for copying machines; marine tooling and composites; heat shields; submarine hulls; prototype generation; development of experimental models; laminated trim; drilling fixtures; bonding jigs; inspection fixtures; industrial metal forming dies; aircraft stretch block and hammer forms; vacuum molding tools; flooring, including flooring for production and assembly areas, clean rooms, machine shops, control rooms, laboratories, parking garages, freezers, coolers, and outdoor loading docks; electrically conductive compositions for antistatic applications; for decorative flooring; expansion joints for bridges; injectable mortars for patch and repair of cracks in structural concrete; grouting for tile; machinery rails; metal dowels; bolts and posts; repair of oil and fuel storage tanks, and numerous other applications.

The compositions and methods described herein are further illustrated by the following non-limiting examples.

### EXAMPLES

The materials listed in Table 1 were used in the examples. Unless specifically indicated otherwise, the amount of each component is in weight percent in the following examples, based on the total weight of the composition.

**Table 1.**

| Component | Description | Source |
|---|---|---|
| PPPBP-epoxy | 1,1-bis(4-epoxyphenyl)-N-phenylphthalimidine, with a melting point of 157-158°C and an epoxy equivalent weight 252.5 grams/equivalent | SABIC |
| NMP | N-methyl pyrrolidone (≥99.5%) | Sigma Aldrich |
| MDI | Methylene diphenyl isocyanate (98%) | Sigma Aldrich |
| TBAB | Tetrabutyl ammonium bromide (≥98%) | Sigma Aldrich |
| DDS | 4,4'-Diaminodiphenyl sulfone (bis(4-aminophenyl sulfone)) (≥98%) | Acros |
| NMA | Methyl-5-norbornene-2,3-dicarboxylic anhydride (nadic methyl anhydride) | Sigma Aldrich |

Melting point (Tₘ) was determined by differential scanning calorimetry (DSC) according to ASTM D3418. A polymer sample (10 to 20 mg) was heated from 40 to 300°C (20°C/min), held at 300°C for 1 min, cooled to 40°C (20°C /min), then held at 40°C for 1 min, and the above heating/cooling cycle was repeated. The second heating cycle is used to obtain the Tₘ.

Glass transition temperature (T_{g}) was determined by DSC according to ASTM D3418. In a typical procedure, a polymer sample (10 to 20 mg) was heated from 0 to 300°C (20°C /min).

Degradation temperature (T_{d}) was determined using thermogravimetric analysis (TGA). Thermogravimetric analysis was performed in a TGA Q5000 instrument according to ASTM E1131 under nitrogen atmosphere from 25°C to 800°C (20°C /min).

Proton nuclear magnetic resonance spectroscopy (¹H NMR) was determined using an Agilent 600 MHz NMR fitted with a 5 mm OneNMR probe in CDCl₃.

Number average molecular weight (Mn) was determined by gel permeation chromatography (GPC), using Agilent EasiVials PS-M standard solution as standards and chloroform as an eluant. Weight average molecular weight (Mw) was determined by GPC under similar conditions.

A general procedure for preparation of the thermosetting oligomers is depicted in Scheme 1 below, wherein n is the number of repeat units as described above.

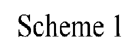

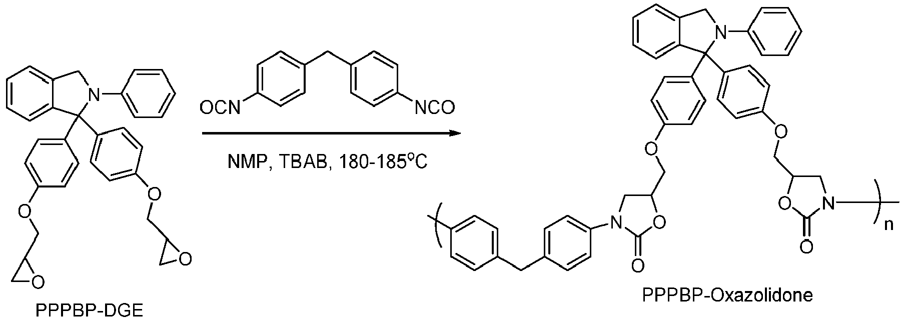

To a three necked flask was fitted with a condenser, magnetic stirrer and pressure equalizing dropping funnel, PPPBP-diglycidyl ether (PPPBP-DGE) (10 g, 20 mmol) was added followed by tetrabutylammonium bromide (TBAB) (0.13 g, 0.2% by weight of the combined monomers). N-methyl pyrrolidone (NMP, 30 mL) was added with the aid of a syringe and the reaction mixture stirred while being flushed with dry nitrogen gas for one hour. The temperature was gradually raised with continuous stirring to 180-185°C, using an oil bath. Diisocyanate (5 g, 20 mmol) dissolved in 20 mL of NMP was added dropwise over half an hour. The solution was maintained at 180-185°C for 5 hours and then cooled gradually. The viscous mixture from the flask was poured into 1000 ml of dry, distilled methanol and vigorously stirred for 30 minutes. A colloidal suspension appeared and a few drops of saturated NH₄Cl in methanol were added while stirring. The precipitate observed was filtered and dried under vacuum in an oven at 80°C for six hours.

A general procedure for cure of the polyoxazolidone-phthalimidine oligomers is a follows. A sample of a polyoxazolidone oligomer, bisphenol A-diglycidyl ether (BPA-DGE) and either DDS or NMA as a curing promoter were dissolved with heating. When the solution became homogeneous, it was transferred to an aluminum pan and placed in oven (pre-heated to 120°C) and the samples were cured according to the cure protocol of Table 2.

**Table 2.**

| | Cure temperature (°C) | Cure time (min) |
|---|---|---|
| Step 1 | 150 | 30 |
| Step 2 | 180 | 30 |
| Step 3 | 210 | 30 |

### Examples 1-4

In order to prepare oligomers containing polyoxazolidone moieties instead of isocyanurate moieties, an equimolar or lower ratio of isocyanate to epoxy groups were used. The ratio of isocyanate (MDI) to epoxy moieties (PPPBP-DGE) was varied from 1:1 to 1:2 as shown in Table 3 to obtain the polyoxazolidone moieties with or without epoxy groups. Table 3 further shows the properties of the oligomers.

**Table 3.**

| Component | Ex. 1 | Ex. 2 | Ex. 3 | Ex. 4 |
|---|---|---|---|---|
| PPPBP-DGE (g) | 10 | 10 | 10 | 10 |
| NMP (mL) | 30 mL | 30 | 30 | 30 |
| MDI (g) | 5 | 4.5 | 3.32 | 2.48 |
| TBAB (g) | 0.013 | 0.13 | 0.13 | 0.13 |
| Mole ratio of isocyanate : epoxy | 1 : 1 | 1 : 1.1 | 1 : 1.5 | 1 : 2 |

| Properties | | | | |
|---|---|---|---|---|
| Mn (Da) | 756 | 1476 | 1732 | 1425 |
| Mw (Da) | 2972 | 5096 | 4418 | 3139 |
| PDI | 3.9 | 3.5 | 2.5 | 2.15 |
| Td (degradation temp) | 373.5 | | | |
| Tg (DSC) | 140°C | 154°C | 190°C | 175°C |

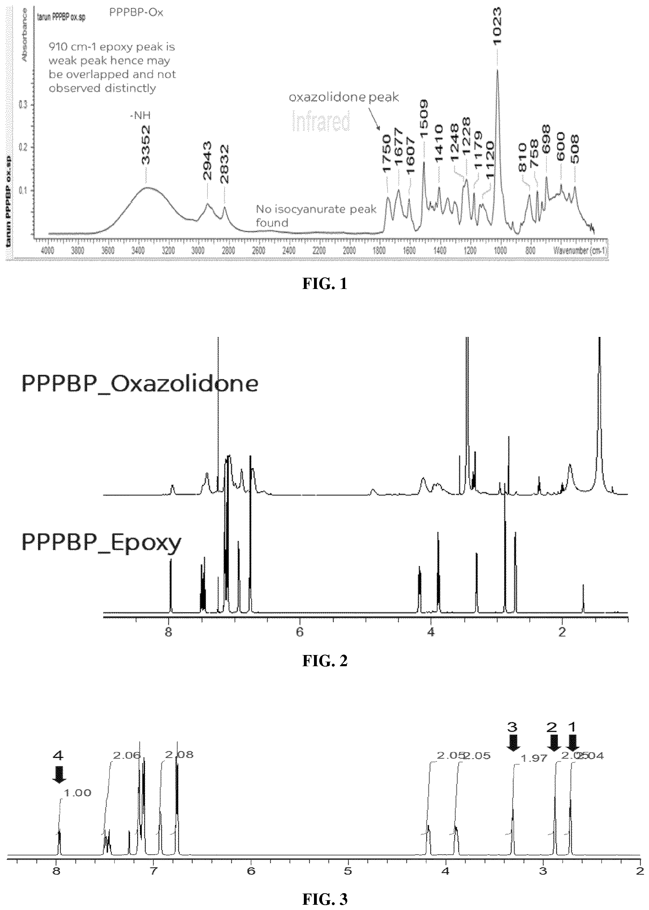

Infrared spectroscopy was performed on Ex. 1 (FIG. 1). FIG. 1 shows that no isocyanate, or isocyanurate group is present, but that oxazolidone group is very strongly present. It was further found that a very high epoxy equivalent weight (EEW) could be detected by ¹H NMR (FIG. 2).

In Example 2, the ratio of MDI to PPPBP-DGE was 1:1.1, to obtain epoxy-terminated oligomers. Multiple samples were taken during 5-hour reaction time, (i.e., at 0.5, 1, 2, 3, 4, and 5 hours) to understand the reaction kinetics of the reaction. Analysis by ¹H NMR (FIG. 3) shows that epoxy group was present (FIG. 3) after 5 hr reaction time. Certain peaks are indicated (1-4). The epoxy protons are labelled 1-3. Protons corresponding to the PPPBP-oxazolidone are labelled as 4.

These ¹H NMR spectra show that the reaction was essentially completed within half an hour, although there was a slight change in the number of epoxy groups over a longer time. This was further supported by the GPC data of Table 4, where it can be seen that maximum Mn generation is achieved within half an hour of starting the reaction.

**Table 4. Example 2**

| Time | Mn (Da) | Mw (Da) | PDI |
|---|---|---|---|
| 0.5 | 1590 | 3588 | 2.2 |
| 1 hr | 1477 | 3935 | 2.7 |
| 2 hr | 1415 | 4534 | 3.3 |
| 3 hr | 1334 | 5065 | 3.6 |
| 4 hr | 1551 | 5355 | 3.5 |
| 5 hr | 1476 | 5096 | 3.5 |

In Example 3, the ratio of MDI to PPPBP-DGE was 1:1.5. This reaction was also continued for 5 hours and samples were taken at 0.5 hours, 1 hour, 3 hours, and 5 hours. ¹H NMR data (spectrum not shown) were analyzed as in Example 2, and again shows that reaction was completed within half an hour of reaction. Epoxy content with respect to PPPBP moiety was followed throughout the reaction time. As can be seen from Table 5, epoxy proton signals were gradually diminishing with time, indicating a reaction between epoxy groups of PPPBP-DGE with isocyanates of MDI.

**Table 5. Example 3**

| Time (hr) | PPPBP protons: Epoxy protons |
|---|---|
| 0 | 1:2 |
| 0.5 | 1:0.49 |
| 0.5 (water-washed) | 1:0.8 |
| 1.0 | 1:0.5 |
| 3.0 | 1:0.34 |
| 5.0 | 1:0.32 |

In Example 4, the ratio of MDI to PPPBP-DGE was 1:2. An increase in PPPBP-DGE content resulted in a decrease in Mw with insignificant change in Mn. The Tg of the composition was found to be 175°C.

### Examples 5-7

A sample of the thermosetting phthalimidine-polyoxazolidone oligomer as made above (containing DDS but no BPA-DGE) was subjected to heating at 220°C for 30 mins and cooled (Ex. 5). There was no Tg visible in DSC. Samples were cured in presence of BPA-DGE and a curing promoter (DDS, Example 6, or NMA, Example 7) at 150, 180, and 210°C (each for 30 mins). Samples were cooled and Tg was measured. Details of the properties of the cured sample are shown in Table 6.

**Table 6.**

| Ex. No. | Weight of oligomer (g) | BPA-DGE (g) | Curing promoter (g) | EEW* | Tg |
|---|---|---|---|---|---|
| 5 | 0.2 | - | 0.01 (DDS) | 2102 | No Tg |
| 6 | 0.2 | 0.8 | 0.5 (DDS) | 2102 | 152°C |
| 7 | 0.3 | 0.7 | 0.32 (NMA) | 2102 | 170°C |

| | | | | | |
|---|---|---|---|---|---|
| *EEW of the phthalimidine-polyoxazolidone oligomer before cure | | | | | |

This disclosure further encompasses the following aspects.
Aspect 1: A thermosetting oligomer comprising repeat units of the formula 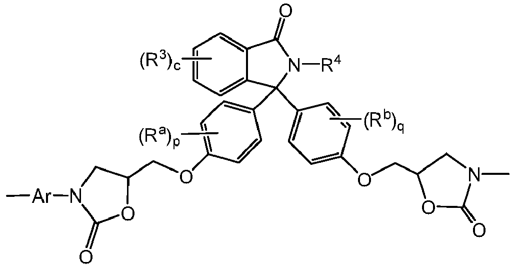 wherein Ar is a C₆₋₃₂ arylene, preferably a C₁₃₋₁₇ diarylene methylene; R^{a} and R^{b} at each occurrence are each independently a halogen, C₁₋₁₂ alkyl, C₂₋₁₂ alkenyl, C₃₋₈ cycloalkyl, or C₁₋₁₂ alkoxy, preferably a C₁₋₃ alkyl; R³ at each occurrence is independently a halogen or a C₁₋₆ alkyl group, preferably a C₁₋₃ alkyl;R⁴ a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; and c, p and q at each occurrence are each independently 0 to 4.
Aspect 2: The thermosetting oligomer of aspect 1, comprising repeat units of the formula 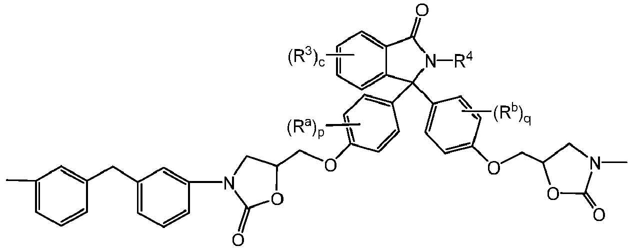 wherein R^{a}, R^{b}, R³, R⁴, c, p, and q are as defined in aspect 1.
Aspect 3: The thermosetting oligomer of aspects 1 or 2, comprising repeat units of the formula 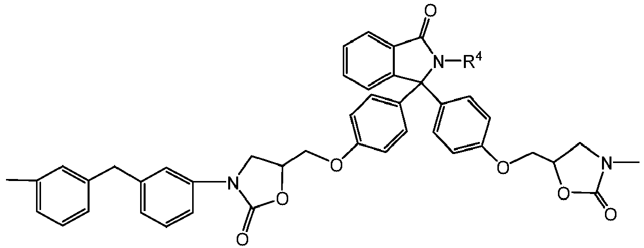 wherein R⁴ is methyl or phenyl, preferably phenyl.
Aspect 4: The thermosetting oligomer of any one or more of aspects 1 to 3, wherein the oligomer is epoxy-group terminated.
Aspect 5: A thermosetting composition comprising the thermosetting oligomer of any one or more of aspects 1 to 4; a curing promoter; and optionally, an auxiliary compound co-curable with the thermoset oligomer.
Aspect 6: The thermosetting composition of aspect 5, wherein the curing promoter is an aliphatic amine, a cycloaliphatic amine, an amine-functional adduct with an epoxy resin, a Mannich base, an aromatic amine, a polyamide, an amidoamine, a phenalkamine, a dicyandiamide, a polycarboxylic acid-functional polyester, a carboxylic acid anhydride, an amine-formaldehyde resins, a phenol-formaldehyde resin, a polysulfide, a polymercaptan, a tertiary amine, a Lewis acid, a latent cationic cure catalyst, or a combination thereof; preferably an amine compound, an aromatic dianhydride, or a bicyclic anhydride, or a combination thereof; more preferably wherein the curing promoter is 4,4'-diamino diphenyl sulfone, 4,4'-methylenebis-(2,6-diethylaniline), 4,4'- methylenedianiline, diethyltoluenediamine, 4,4'-methylenebis-(2,6-dimethylaniline), m-phenylenediamine, p-phenylenediamine, 2,4-bis(p-aminobenzyl)aniline, 3,5-diethyltoluene-2,4-diamine, 3,5-diethyltoluene-2,6-diamine, m-xylylenediamine, p-xylylenediamine, diethyl toluene diamines, methyl-5-norbornene-2,3-dicarboxylic anhydride, or a combination thereof; most preferably wherein the curing promoter is 4,4'-diamino diphenyl sulfone, 4,4'-methylenebis-(2,6-diethylaniline), methyl-5-norbornene-2,3-dicarboxylic anhydride, or a combination thereof.
Aspect 7: The thermosetting composition of aspect 5 or aspect 6, wherein the co-curable auxiliary compound comprises an auxiliary epoxy compound, bifunctional epoxy resin , multifunctional epoxy resin, cycloaliphatic epoxy resin, aromatic epoxy resin, or a combination thereof; preferably wherein the auxiliary epoxy compound is an aliphatic epoxy compound, cycloaliphatic epoxy compound, aromatic epoxy compound, bisphenol A epoxy compound, bisphenol F epoxy compound, phenol novolac epoxy polymer, cresol-novolac epoxy polymer, biphenyl epoxy compound, triglycidyl p-aminophenol, tetraglycidyl diamino diphenyl methane, naphthalene epoxy compound, divinylbenzene dioxide compound, 2-glycidylphenylglycidyl ether, dicyclopentadiene epoxy compound, multi-aromatic epoxy polymer, bisphenol S epoxy compound, isocyanurate epoxy compound, hydantoin epoxy compound, or a combination thereof; more preferably wherein the auxiliary epoxy compound is a bisphenol A diglycidylether, a bisphenol F diglycidylether, a neopentylglycol diglycidyl ether, a 3,4-epoxycyclohexylmethyl-3,4-epoxycyclohexane carboxylate, a *N,N*-diglycidyl-4-glycidyloxyaniline, a N,N,N',N'-tetraglycidyl-4,4'-diaminodiphenylmethane, or a combination thereof.
Aspect 8: The thermosetting composition of any one or more of aspects 5 to 7, further comprising an additive, wherein the additive is a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof.
Aspect 9: A thermoset, comprising the cured thermosetting composition of any one or more of aspects 5 to 8, optionally where the thermoset has a glass transition temperature of greater than or equal to 80°C, preferably greater than or equal to 100°C, and more preferably greater than or equal to 140°C..
Aspect 10: An article comprising the cured thermoset of aspect 9, preferably wherein the article is a composite, a foam, a fiber, a layer, a coating, an encapsulant, an adhesive, a sealant, a sizing resin, a prepreg, a powder coating, a casing, a component, or a combination thereof.
Aspect 11: A method for the manufacture of a thermoset, the method comprising polymerizing and crosslinking the epoxy composition of any one or more of aspects 5 to 8.
Aspect 12: A method for the manufacture of the thermosetting oligomer of any one or more of aspects 1 to 4, comprising reacting an arylene diisocyanate and a diepoxy phthalimidine of the formula 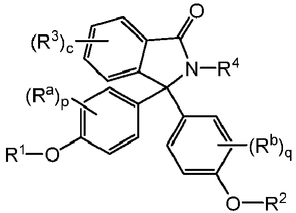 wherein R¹ and R² are each independently an epoxide-containing functional group, preferably wherein R¹ and R² at each occurrence are each independently of the formula 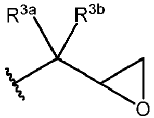 wherein R^{3a} and R^{3b} are each independently hydrogen or C₁₋₁₂ alkyl, more preferably wherein R^{3a} and R^{3b} are each hydrogen; and R^{a}, R^{b}, R³, R⁴, c, p, and q are as defined in aspect 1.
Aspect 13: The method of aspect 12, wherein the diepoxy phthalimidine has a purity of greater than 60%, preferably greater than 70%, preferably greater than 80%, preferably greater than 90%, preferably greater than 95%, preferably greater than 98%, and preferably greater than 99% as determined by high performance liquid chromatography, or an epoxy equivalent weight of 1.0 to 1.5, preferably 1.0 to 1.2, more preferably 1.0 to 1.15, even more preferably 1.0 to 1.1 times relative to the theoretical epoxy equivalent weight, or a combination of the purity and epoxy equivalent weight.
Aspect 14: The method of aspect 12 or 13, wherein the reaction is in the presence of a catalyst, an organic solvent, or both, preferably wherein the reaction is in the presence an ammonium or phosphonium catalyst and a polar organic solvent.
Aspect 15: The method of any one or more of aspects 12 to 14, wherein a mole ratio of the arylene isocyanate to the diepoxy phthalimidine is from 1:1 to 1:2.

The compositions, methods, and articles disclosed herein can alternatively comprise, consist of, or consist essentially of, any appropriate components or steps herein disclosed. The compositions, methods, and articles can additionally, or alternatively, be formulated so as to be devoid, or substantially free, of any steps, components, materials, ingredients, adjuvants, or species that are otherwise not necessary to the achievement of the function and/or objectives of the compositions, methods, and articles.

All ranges disclosed herein are inclusive of the endpoints, and the endpoints are independently combinable with each other. "Combinations" is inclusive of blends, mixtures, alloys, reaction products, and the like. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. Reference throughout the specification to "an aspect" means that a particular element described in connection with the aspect is included in at least one aspect described herein, and may or may not be present in other aspects. The term "or a combination thereof' includes one or more of the named elements, and is open to one or more like elements not named. The described elements may be combined in any suitable manner in the various aspects.

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this invention belongs. All cited patents, patent applications, and other references are incorporated herein by reference in their entirety. While particular aspects have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group.

The term "alkyl" means a branched or straight chain, unsaturated aliphatic hydrocarbon group, e.g., methyl, ethyl, n-propyl, i-propyl, n-butyl, s-butyl, t-butyl, n-pentyl, s-pentyl, and n- and s-hexyl. "Alkenyl" means a straight or branched chain, monovalent hydrocarbon group having at least one carbon-carbon double bond (e.g., ethenyl (-HC=CH₂)). "Alkoxy" means an alkyl group that is linked via an oxygen (i.e., alkyl-O-), for example methoxy, ethoxy, and sec-butyloxy groups. "Alkylene" means a straight or branched chain, saturated, divalent aliphatic hydrocarbon group (e.g., methylene (-CH₂-) or, propylene (-(CH₂)₃-)). "Cycloalkenyl" means a monovalent group having one or more rings and one or more carbon-carbon double bonds in the ring, wherein all ring members are carbon (e.g., cyclopentyl and cyclohexyl). "Aryl" means an aromatic hydrocarbon group containing the specified number of carbon atoms, such as phenyl, tropone, indanyl, or naphthyl. "Arylene" means a divalent aryl group. "Alkylarylene" means an arylene group substituted with an alkyl group. "Arylalkylene" means an alkylene group substituted with an aryl group (e.g., benzyl). The prefix "halo" means a group or compound including one more of a fluoro, chloro, bromo, or iodo substituent. A combination of different halo groups (e.g., bromo and fluoro), or only chloro groups can be present.

Unless substituents are otherwise specifically indicated, each of the foregoing groups can be unsubstituted or substituted, provided that the substitution does not significantly adversely affect synthesis, stability, or use of the compounds or oligomers. "Substituted" means that the compound or group is substituted with at least one (e.g., 1, 2, 3, or 4) substituents that can each independently be a C₁₋₉ alkoxy, a C₁₋₉ haloalkoxy, a nitro (-NO₂), a cyano (-CN), a C₁₋₆ alkyl sulfonyl (-S(=O)₂-alkyl), a C₆₋₁₂ aryl sulfonyl (-S(=O)₂-aryl) a thiol (-SH), a thiocyano (-SCN), a tosyl (CH₃C₆H₄SO₂-), a C₃₋₁₂ cycloalkyl, a C₂₋₁₂ alkenyl, a C₅₋₁₂ cycloalkenyl, a C₆₋₁₂ aryl, a C₇₋₁₃ arylalkylene, a C₄₋₁₂ heterocycloalkyl, and a C₃₋₁₂ heteroaryl instead of hydrogen, provided that the substituted atom's normal valence is not exceeded. The number of carbon atoms indicated in a group is exclusive of any substituents. For example -CH₂CH₂CN is a C₂ alkyl group substituted with a nitrile.

While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A thermosetting oligomer comprising repeat units of the formula 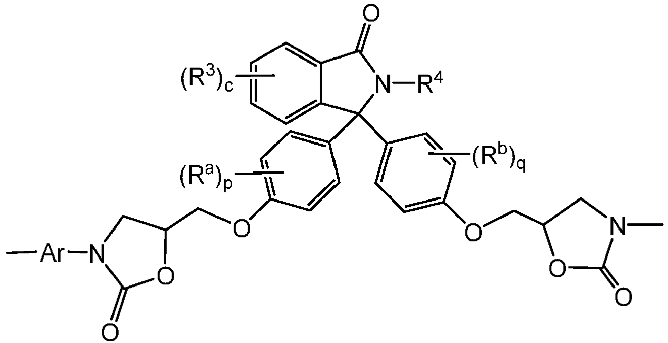 wherein
Ar is a C₆₋₃₂ arylene, preferably a C₁₃₋₁₇ diarylene methylene;
R^{a} and R^{b} at each occurrence are each independently a halogen, C₁₋₁₂ alkyl, C₂₋₁₂ alkenyl, C₃₋₈ cycloalkyl, or C₁₋₁₂ alkoxy, preferably a C₁₋₃ alkyl;
R³ at each occurrence is independently a halogen or a C₁₋₆ alkyl group, preferably a C₁₋₃ alkyl;
R⁴ a C₁₋₂₅ hydrocarbyl, preferably a C₁₋₆ alkyl, a phenyl, or a phenyl substituted with up to five C₁₋₆ alkyl groups, more preferably a C₁₋₃ alkyl or a phenyl; and
c, p and q at each occurrence are each independently 0 to 4.

2. The thermosetting oligomer of claim 1, comprising repeat units of the formula 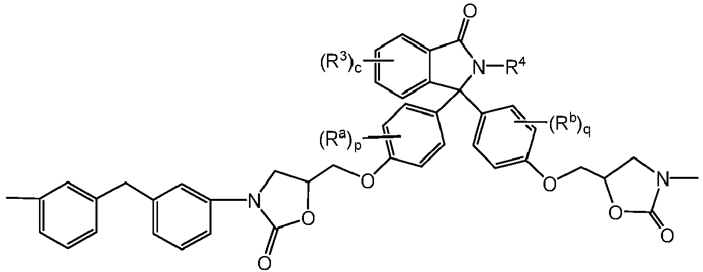 wherein R^{a}, R^{b}, R³, R⁴, c, p, and q are as defined in claim 1.

3. The thermosetting oligomer of claim 1, comprising repeat units of the formula 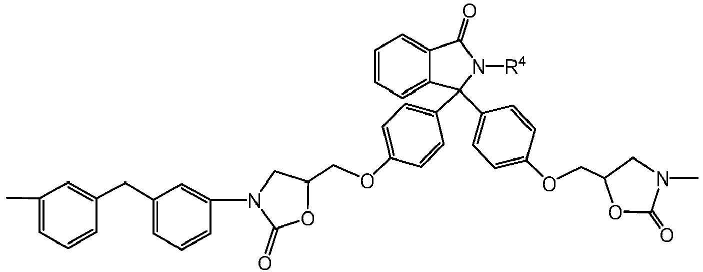 wherein R⁴ is methyl or phenyl, preferably phenyl.

4. The thermosetting oligomer of any one or more of claims 1 to 3, wherein the oligomer is epoxy-group terminated.

5. A thermosetting composition, comprising
the thermosetting oligomer of any one or more of claims 1 to 4;
a curing promoter; and
optionally, an auxiliary compound co-curable with the thermoset oligomer.

6. The thermosetting composition of claim 5,
wherein the curing promoter is aliphatic amine, a cycloaliphatic amine, an amine-functional adduct with an epoxy resin, a Mannich base, an aromatic amine, a polyamide, an amidoamine, a phenalkamine, a dicyandiamide, a polycarboxylic acid-functional polyester, a carboxylic acid anhydride, an amine-formaldehyde resins, a phenol-formaldehyde resin, a polysulfide, a polymercaptan, a tertiary amine, a Lewis acid, a latent cationic cure catalyst, or a combination thereof;
preferably wherein the curing promoter is an amine compound, an aromatic dianhydride, or a bicyclic anhydride, or a combination thereof;
more preferably wherein the curing promoter is 4,4'-diamino diphenyl sulfone, 4,4'-methylenebis-(2,6-diethylaniline), 4,4'- methylenedianiline, diethyltoluenediamine, 4,4'-methylenebis-(2,6-dimethylaniline), m-phenylenediamine, p-phenylenediamine, 2,4-bis(p-aminobenzyl)aniline, 3,5-diethyltoluene-2,4-diamine, 3,5-diethyltoluene-2,6-diamine, m-xylylenediamine, p-xylylenediamine, diethyl toluene diamines, methyl-5-norbornene-2,3-dicarboxylic anhydride, or a combination thereof;
more preferably wherein the curing promoter is 4,4'-diamino diphenyl sulfone, 4,4'-methylenebis-(2,6-diethylaniline), methyl-5-norbornene-2,3-dicarboxylic anhydride, or a combination thereof.

7. The thermosetting composition of claim 5 or claim 6,
wherein the co-curable auxiliary compound comprises an auxiliary epoxy compound, bifunctional epoxy resin, multifunctional epoxy resin, cycloaliphatic epoxy resin, aromatic epoxy resin, or a combination thereof;
preferably wherein the auxiliary epoxy compound is an aliphatic epoxy compound, cycloaliphatic epoxy compound, aromatic epoxy compound, bisphenol A epoxy compound, bisphenol F epoxy compound, phenol novolac epoxy polymer, cresol-novolac epoxy polymer, biphenyl epoxy compound, triglycidyl p-aminophenol, tetraglycidyl diamino diphenyl methane, naphthalene epoxy compound, divinylbenzene dioxide compound, 2-glycidylphenylglycidyl ether, dicyclopentadiene epoxy compound, multi-aromatic epoxy polymer, bisphenol S epoxy compound, isocyanurate epoxy compound, hydantoin epoxy compound, or a combination thereof;
more preferably wherein the auxiliary epoxy compound is a bisphenol A diglycidylether, a bisphenol F diglycidylether, a neopentylglycol diglycidyl ether, a 3,4-epoxycyclohexylmethyl-3,4-epoxycyclohexane carboxylate, a *N,N*-diglycidyl-4-glycidyloxyaniline, a N,N,N',N'-tetraglycidyl-4,4'-diaminodiphenylmethane, or a combination thereof.

8. The thermosetting composition of any one or more of claims 5 to 7, further comprising an additive, wherein the additive is a particulate filler, fibrous filler, antioxidant, heat stabilizer, light stabilizer, ultraviolet light stabilizer, ultraviolet light-absorbing compound, near infrared light-absorbing compound, infrared light-absorbing compound, plasticizer, lubricant, release agent, antistatic agent, anti-fog agent, antimicrobial agent, colorant, surface effect additive, radiation stabilizer, flame retardant, anti-drip agent, fragrance, a polymer different from the thermoset polymer, or a combination thereof.

9. A thermoset, comprising the cured thermosetting composition of any one or more of claims 5 to 8, optionally where the thermoset has a glass transition temperature of greater than or equal to 80°C, preferably greater than or equal to 100°C, and more preferably greater than or equal to 140°C.

10. A method for the manufacture of a thermoset, the method comprising heat curing the thermosetting composition of any one or more of claims 5 to 8.

11. An article comprising the cured thermoset of claim 9, preferably wherein the article is a composite, a foam, a fiber, a layer, a coating, an encapsulant, an adhesive, a sealant, a sizing resin, a prepreg, a powder coating, a casing, a component, or a combination thereof.

12. A method for the manufacture of the thermosetting oligomer of any one or more of claims 1 to 4, comprising reacting an arylene diisocyanate and a diepoxy phthalimidine of the formula 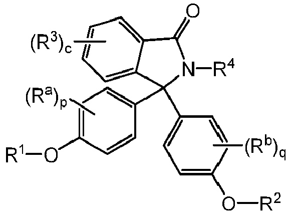 wherein
R¹ and R² are each independently an epoxide-containing functional group, preferably wherein R¹ and R² at each occurrence are each independently of the formula 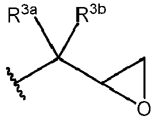 wherein R^{3a} and R^{3b} are each independently hydrogen or C₁₋₁₂ alkyl, more preferably wherein R^{3a} and R^{3b} are each hydrogen; and
R^{a}, R^{b}, R³, R⁴, c, p, and q are as defined in claim 1.

13. The method of claim 12, wherein the diepoxy phthalimidine has
a purity of greater than 60%, preferably greater than 70%, preferably greater than 80%, preferably greater than 90%, preferably greater than 95%, preferably greater than 98%, and preferably greater than 99% as determined by high performance liquid chromatography, or
an epoxy equivalent weight of 1.0 to 1.5, preferably 1.0 to 1.2, more preferably 1.0 to 1.15, even more preferably 1.0 to 1.1 times relative to the theoretical epoxy equivalent weight, or
a combination of the purity and epoxy equivalent weight.

14. The method of claim 12 or 13, wherein the reaction is in the presence of a phase transfer catalyst and optionally an organic solvent, preferably in the presence of an ammonium or phosphonium catalyst and a polar organic solvent.

15. The method of any one or more of claims 12 to 14, wherein a mole ratio of the arylene isocyanate to the diepoxy phthalimidine is from 1:1 to 1:2.
